# EUROPEAN PATENT APPLICATION

(11) **EP 1 298 572 A2**
(43) Date of publication of application: **02.04.2003**
(21) Application number: 02021774.1
(22) Date of filing: 26.09.2002
(51) Int. Cl.: G06F 19/00

(54) **Method for analyzing trait map**

(30) Priority: 26.09.2001 JP 2001294013
(71) Applicant: RIKEN, Wako-shi, Saitama 351-0198 (JP)
(72) Inventor: Toyoda, Tetsuro, Riken Yokohama Institute, Yokohama-shi, Kanagawa, 230-0045 (JP)
(74) Representative: Sternagel, Fleischer, Godemeyer & Partner Patentanwälte

(57) **Abstract**

A method which provides a user who operates a computer with information on bio-molecular connection, the method comprises:
(1) step wherein the user selects two or more intervals on a genomic coordinate by a computer operation;
(2) step of generating a datum which shares one or more identifiers of bio-molecules with all of the selected intervals in step (1) based on one or more records stored in a database; and
(3) step of providing the use with the generated datum as information on bio-molecular connection.

## Description

### Technical Field

The present invention relates to a data processing system for an analysis of a trait map.

### Related Art

A quantitative trait such as blood sugar level or body height is considered to be controlled by a combination effect of multiple genetic factors(epistasis). A gene locus which participates in this quantitative trait is called QTL (Quantitative Trait Locus). Recently, for a purpose of taking the effect of epistasis into QTL analysis, an analysis for mapping trait has been carried out considering a combination of alleles at 2 or more marker gene loci (called marker alleles).

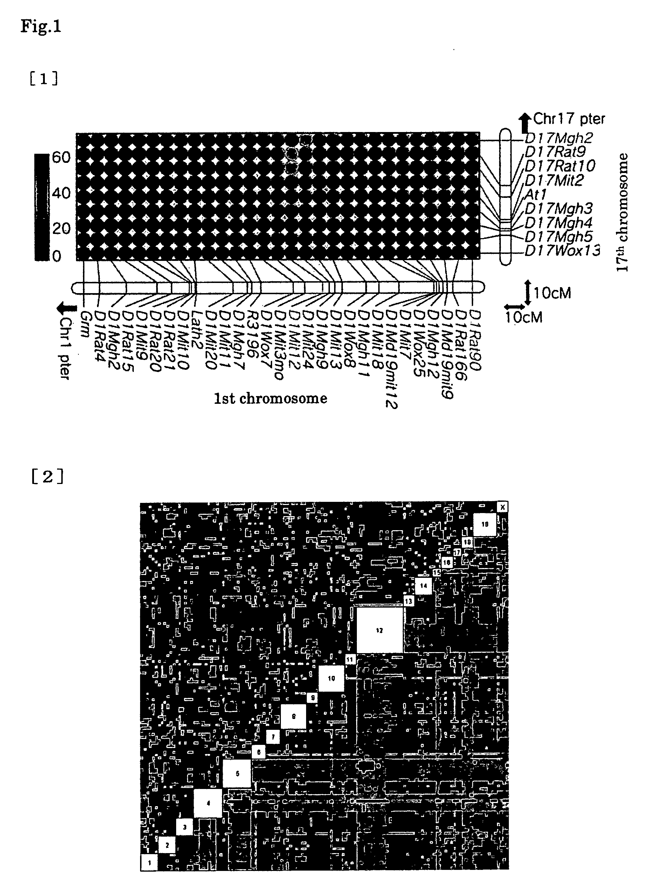

For example, Fig.1 [1] depicts a degree of correlation between phenotypes and marker alleles using an OLETF noninsulin dependent diabetes model rats (Kenichi Matsubara · Yoshiyuki Sakaki, eds., Genome Information Biology, Chapter II QTL Analysis, Nakayama Shoten, 2000). In the figure, an intensity of influence in blood sugar level is evaluated by F test considering the combination of marker alleles at the 1st chromosome and the 17th chromosome of rats, and significances are displayed in colors. It is thus possible to display degrees of correlation between phenotypes and marker alleles at 2 marker gene loci by using a two dimensional genomic coordinate system.

As other example, Fig.1 [2] depicts mapping of quantitative traits on the entire genome of mice based on a circadian rhythm as a trait (Shimomura *et al.* "Genome-Wide Epistatic Interaction Analysis Reveals Complex Genetic Determinants of Circadian Behavior in Mice," Genome Research, 2001, vol. 11, 959-980. In the figure, a genomic coordinate, in which 1st to 19th chromosomes and X chromosome of mice are connected, is employed, and the intensity of epistasis between 2 marker gene loci is displayed in colors. Intensity of epistasis is not calculated between the marker gene loci in the same chromosome, which is shown as blanks, and analytical techniques employed for upper left and lower right are different. Accordingly, it has become possible to express a degree of correlation between phenotypes and marker alleles in 2 marker gene loci by using a two dimensional genomic coordinates system. In the specification, Figs. 1 [1] and [2] are referred to as "trait maps".

Conventional trait maps created by genetics techniques such as QTL analysis are suitable to give an overview of epistasis between marker gene loci. However, an information system has not been known in which a viewer of a trait map is able to search and view candidate causative genes for the trait of interest from a database with a simple operation. Moreover, the viewer of the trait map is not able to select candidate causative genes by relating the trait map to information on bio-molecular connection at molecular level under an interactive operation. Therefore, an enormous burden is required to use a trait map, and for this reason, many researchers have not been in condition of conveniently utilizing trait maps for progressing researches. In particular, lots of areas with high degree of correlation between phenotypes and marker alleles (in the specification, such areas is referred to as "peaks") are observed in many analyses. Therefore, a development of an information system has been desired earnestly which enables easy selection and analysis of each peak under an interactive operation.

### Disclosure of the Invention

An object of the present invention is to provide a method for analyzing a trait map. More specifically, the object of the present invention is to provide a method for analyzing a trait map in which a viewer of a trait map is able to search and view information related to candidate causative genes for the trait with a simple operation. Selection of plural gene loci is essential for an analysis considering epistasis, however, each selection of an interval on a genomic coordinate, where each gene locus exists, is much troublesome for a viewer. Therefore, another object of the present invention is to provide a means which enables a viewer to select multiple intervals on the genomic coordinates at the same time and obtain molecular level information immediately.

Furthermore, it is desirable that many researchers are able to utilize an analytical system of a trait map in their own laboratories. Since an amount of information on genes necessary for the analysis is extravagant, and corrections and revisions are progressing in every seconds, it is desirable that such information is maintained centrally at one site. Consequently, providing a constitution of a system meet the above requirements is further object of the present invention.

The inventor noted that, if a system is so constituted as to display candidate causative genes immediately when a viewer of a trait map selects an area on said map with a mouse operation, it will be easy to understand many peaks on the trait map by connecting with molecular level information. The inventor thus constituted a system so that information on bio-molecular connection is displayed depending on selection of each area by a viewer, and found that the system was an effective means to analyze the trait map.

More specifically, the inventor found that, when a trait map is displayed on a monitor of a local computer and then a viewer is lead to select an area on the trait map by operating an input device such as a mouse connected to the local computer, and when a system is constituted so that data, or existence of data, sharing 1 or more identifiers of bio-molecules with all intervals on genomic coordinates corresponding to said area, are immediately displayed after the selection of the area, many peaks on the trait map are easily understandable in connection with molecular level information.

The inventor also found that, when a system is constituted in which an operation from a selection of an area to a display of candidate causative genes is made easy by displaying information on connection of bio-molecules with 1 to 3 clicks including the selection of an area, preferably 1 or 2 clicks, further preferably 1 click and/or mouse over, each peak is understandable in rapid connection with molecular level information, even though many peaks exist, and easy judgment can be made as to whether or not the peak is important. The present invention was achieved on the basis of these findings.

The present invention thus relates to a method of providing a user who operates a computer with information on bio-molecular connection, which comprises the steps of:
(1) a step wherein a user selects two or more intervals on genomic coordinates by a computer operation;
(2) a step of generating a datum which shares one or more identifiers of bio-molecules with all of the intervals selected in step (1) based on one or more records stored in a database; and
(3) a step of presenting the aforementioned generated data to the user as the information on bio-molecular connection. According to a preferred embodiment of the present invention, the aforementioned computer is a local computer in an organization wherein plural computers are connected by a network or networks.

The present invention also provides a method for analyzing a trait map which comprises the aforementioned steps (1) to (3).

According to preferred embodiments of the above inventions, provided are:
the aforementioned method wherein an input program which enables simultaneous selection of two or more intervals is used in a local computer;
the aforementioned method wherein a gene locus space is displayed by assigning genomic coordinates to each axis of two- or three-dimensional orthogonal coordinates system, and a user uses an input program which enables the user to select simultaneously all of intervals which correspond to an area in the gene locus space by selecting the area on the display;
the aforementioned method wherein a degree of correlation between phenotypes and marker alleles is displayed on the locus space;
the aforementioned method wherein the information on bio-molecular connection comprises one or more connection data;
the aforementioned method wherein the user is able to select each connection data by displaying two or more connection data in an order of high priority, and a program for presentation is used in which the user is able to view the selected connection data;
the aforementioned method wherein a program for presentation is used by which a color of a character string representing an identifier of the bio-molecule or a background color of said character string is displayed depending on an expression amount of an intracellular messenger RNA of the identifier of the bio-molecule; and
the aforementioned method wherein a program for presentation is used in a process of presentation, in which a character string representing the identifier of the bio-molecule which is hit in keyword search or homology search is displayed with highlight.

From further aspect of the present invention, provided are:
a program used to conduct the aforementioned methods by computer;
a media which stores a program used to conduct the aforementioned methods by computer;
a computer wherein a program is installed which is used to conduct the aforementioned methods by the computer;
a remote computer used to conduct the aforementioned methods;
a local computer used to conduct the aforementioned methods; and
a database used to conduct the aforementioned methods by a computer.

### Brief Explanation of Drawings

Fig. 1 depicts an example displaying a trait map in a gene locus space. In the figure, [1] shows an example wherein the 1st chromosome and the 17th chromosome of rats are corresponded to the genomic coordinates, [2] shows an example wherein the entire chromosomes of mice are connected and corresponded to the genomic coordinates.

Fig. 2 explains the genomic coordinates. In the figure, [1] shows an example where relative locations of gene loci, L1, L2, and L3 are expressed by a genomic coordinate, [2] shows an example where locations of gene loci are expressed by corresponding one chromosome to one genomic coordinate, [3] shows an example where locations of gene loci is expressed by corresponding one part of a chromosome to one genomic coordinate, and [4] shows an example where locations of gene loci is expressed by corresponding plural chromosomes to one genomic coordinate.

Fig. 3 depicts a unit of the genomic coordinate. In the figure, [1] shows an example where a physical distance (Mb: megabase) is used as a unit expressing locations of gene loci, [2] shows an example where a genetic distance (cM: centimorgan) is used as a unit expressing locations of gene loci, and [3] shows an example where an order of markers is used as a unit expressing locations of gene loci.

Fig. 4 depicts a relation between genomic coordinates and a locus space. In the figure, [1] shows a locus space wherein genomic coordinates are assigned to each axis of a two-dimensional orthogonal coordinate system, and [2] shows a locus space wherein genomic coordinates are assigned to each axis of a tree-dimensional orthogonal coordinate system.

Fig. 5 depicts an example where a trait map is displayed in a locus space. In the figure, each of [1] and [2] shows a presentation example. Also in the figure, intensities of correlation between phenotypes and marker alleles is expressed with tones and shades and displayed in the locus space.

Fig. 6 depicts a method of selecting plural intervals at the same time by selection of an area in a locus space. In the figure, [1] shows an example where a rectangle is selected by a mouse, and [2] shows an example where a rectangle is selected in a locus space indicating a trait map.

Fig. 7 depicts a method of selecting plural intervals at the same time by selecting an area in a locus space. In the figure, [1] is an example where 1 point is selected by a mouse, and [2] shows an example where 1 point is selected by a mouse in a locus space showing a trait map.

Fig. 8 depicts an example where a trait map is displayed in a locus space. In the figure, [1] shows how an area in a two-dimensional locus space is selected by a mouse, and [2] shows how an area in a three-dimensional locus space is selected by a mouse.

Fig. 9 depicts an organization of computers connected by a network in a system of this invention.

Fig. 10 depicts a flow of information in a system of this invention.

Fig. 11 depicts a method of selecting identifiers of genes whose gene loci exist in a selected interval. In the figure, [1] shows an example where an offset is not used in an interval with a width, [2] shows an example where an offset is further used in an interval with a width, and [3] shows an example where an offset is further used in an interval without width.

Fig. 12 depicts an example of "information on bio-molecular connection." In the figure, [1] shows that an information on bio-molecular connection shares at least 1 or more identifiers of bio-molecules with all of selected intervals, and [2] shows that when an identifier of bio-molecule is shared with only one side of the intervals, the information does not fall under the category of the" information on bio-molecular connection" in this specification.

Fig. 13 depicts an example of connection data.

Fig. 14 depicts a presentation example of trait maps by an input program.

Fig. 15 depicts an example of the information on bio-molecular connection which consists of three connection data.

Fig. 16 depicts a determination method of priority of connection data.

Fig. 17 depicts an example of a user interface of a program for presentation.

Fig. 18 depicts an example where a detailed information is displayed by clicking an identifier.

Fig. 19 depicts an example where a background color of an identifier is colored depending on an expression amount of a gene. In the figure, [1] shows a process where a submenu is displayed by aright click and select "color by expression amount", and [2] shows a process where a background color of an identifier in a path view is colored.

Fig. 20 depicts an example where an identifier hit by a keyword search is highlighted. In the figure, [1] shows a process where a submenu is displayed by a right click and designate "kinase" as a keyword, and [2] shows a process where the hit identifier is indicated in boldface and is flashing.

### Best Mode for Carrying out the Invention

The meanings of the terms used in the specification are as follows.

"Bio-molecule" is a polymer existing in a living organism or one part of the polymer, which includes a polymer comprising an amino acid sequence such as a protein or a polypeptide, or a polymer comprising a nucleic acid sequence such as DNA, RNA, or polynucleotides. A gene coded in a genome, an open reading frame, or an exon is also a bio-molecule. In the specification, data expressing a bio-molecule is regarded to be encompassed within the bio-molecule. Therefore, data on amino acid sequence and those on nucleic acid sequence are also bio-molecules, and a tree-dimensional structure of a protein falls within a bio-molecules.

"Information on bio-molecular connection" is a datum which shares one or more identifiers of bio-molecules with all of selected intervals in step (1) of the method of the present invention, which concept will be further detailed later.

"Identifier" is a name given to an object which is expressible by a datum, and is a unique name which is one-to-one correspondence to said object in a system. Examples of the identifier include "accession" or "PDB (Protein Data Bank) name."

"Gene locus" is a location where a gene is coded on a chromosome. Usually, a gene locus is a region on a chromosome to be transcribed to a continuous poly RNA chain by RNA polymerase, however, the term "a gene locus" is sometimes used to include a region regulating transcription. Furthermore, a region consisting of exons which code a single protein and introns between the exons is sometimes referred to as a gene locus. At least, any information expressing an existing location of a gene or a marker on a chromosome falls within the gene locus used in the specification.

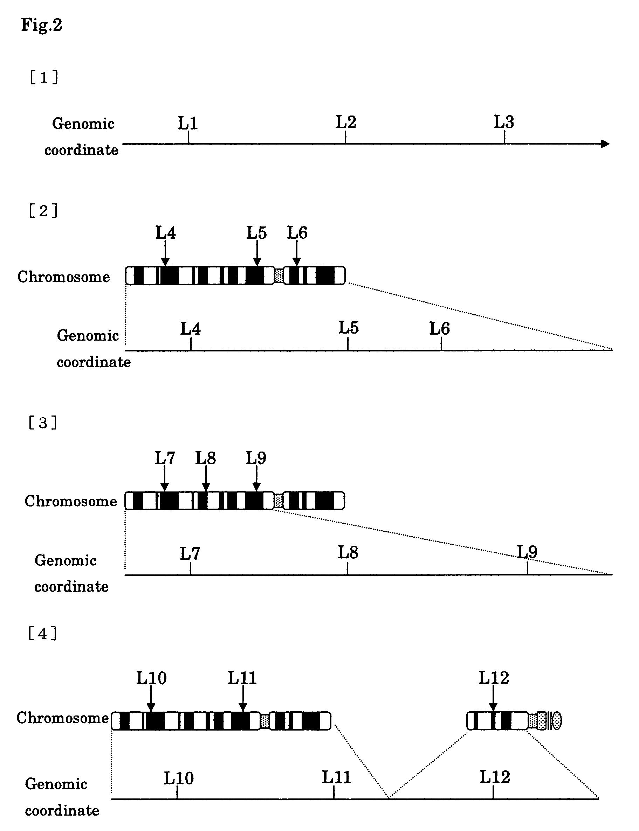

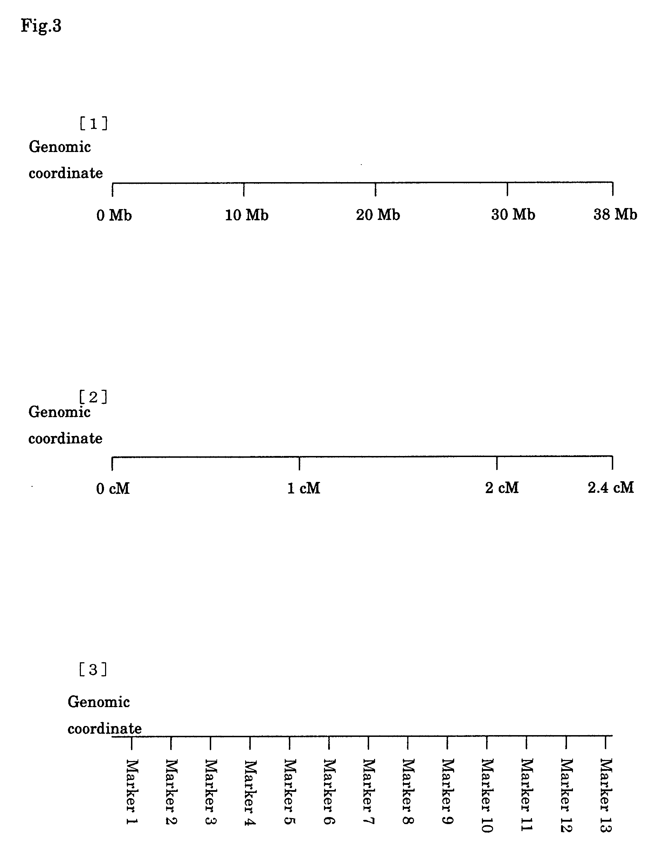

"Genomic coordinate" is one dimensional coordinate used to express relative positions between gene loci on a chromosome, expressing the positions in a direction from 5' terminal to 3' terminal (or in a direction from 3' terminal to 5' terminal) in one of the chains of a double-stranded DNA constituting a chromosome. As shown in Fig. 2 [2], locations of gene loci are sometimes expressed by corresponding one chromosome to one genomic coordinate. Also shown in Fig. 2 [3], locations of gene loci are sometimes expressed by corresponding one part of a chromosome to one genomic coordinate. Moreover, as shown in Fig 2 [4], locations of gene loci are sometimes expressed by connecting multiple chromosome terminals and corresponding to one genomic coordinate. A unit of genomic coordinate is expressed by, for example, a physical distance (such as number of bases) as shown in Fig. 3 [1], genetic distance (such as centimorgan) as shown in Fig. 3 [2], or an order of a marker (for example, markers are placed with equal intervals based on the order on the chromosome) as shown in Fig. 3 [3]. Any unit may be used as long as relative positions between gene loci are expressed accurately.

"Interval on genomic coordinate" is a segment or a point on the genomic coordinate. Its starting point and end point are specified by positions on the genomic coordinate. The starting point and the end point can be expressed by coordinates based on a physical distance, and also can be expressed by coordinates based on a genetic distance. Furthermore, the starting point and the end point can be expressed by 2 markers, or it is possible to express the starting point and the end point by only a single marker.

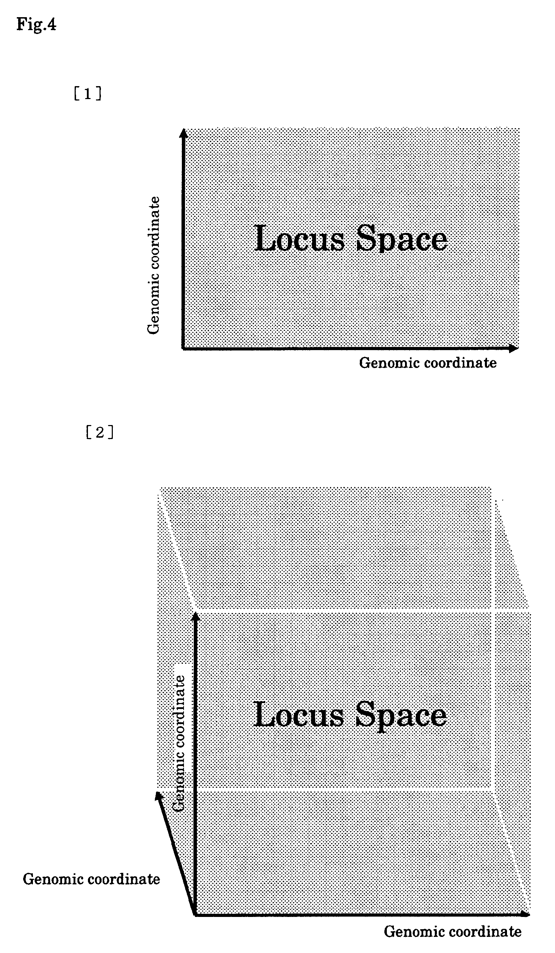

"Assigning genomic coordinates to each axis of an orthogonal coordinates system" means to construct coordinates system as shown in Fig. 4. More specifically, examples are shown where genomic coordinates is assigned to each axis of a two-dimensional orthogonal coordinate system (Fig. 4 [1]), and where genomic coordinates is assigned to each axis of a three-dimensional orthogonal coordinates (Fig. 4 [2]).

"Gene locus space" or "locus space" is a space defined by genomic coordinates assigned to each axis of the orthogonal coordinate system as shown in Fig. 4.

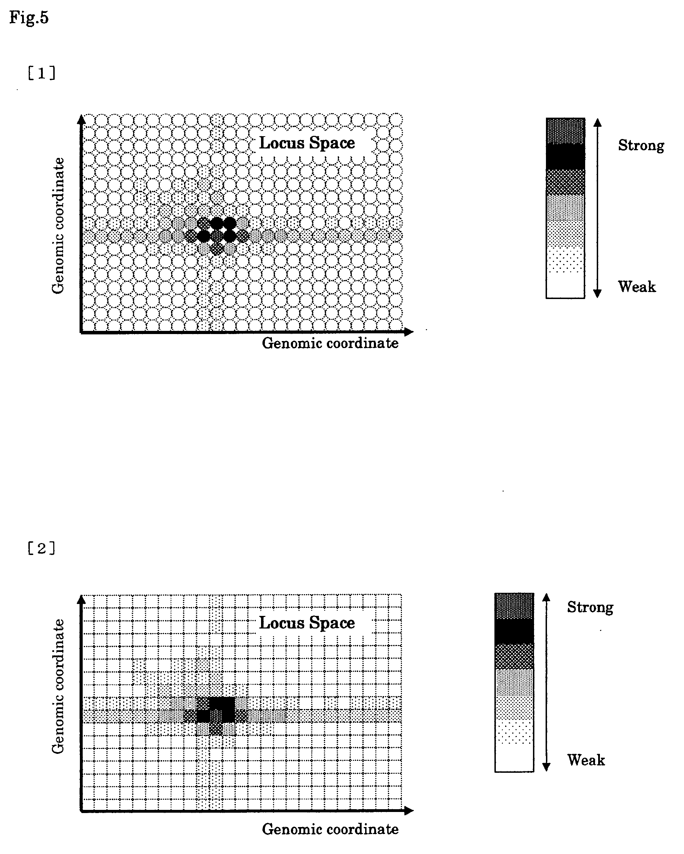

"A degree of correlation between phenotypes and marker alleles in a gene locus space" is often expressed by LOD score, p-value or F-value, and is a preferable mode of displaying a trait map in a gene locus space. As shown in Fig. 5, it is visually readily understandable when the degree of correlation is presented with colors and tints in a gene locus space. Fig. 1 depicts an example of presentation of a trait map in a gene locus space.

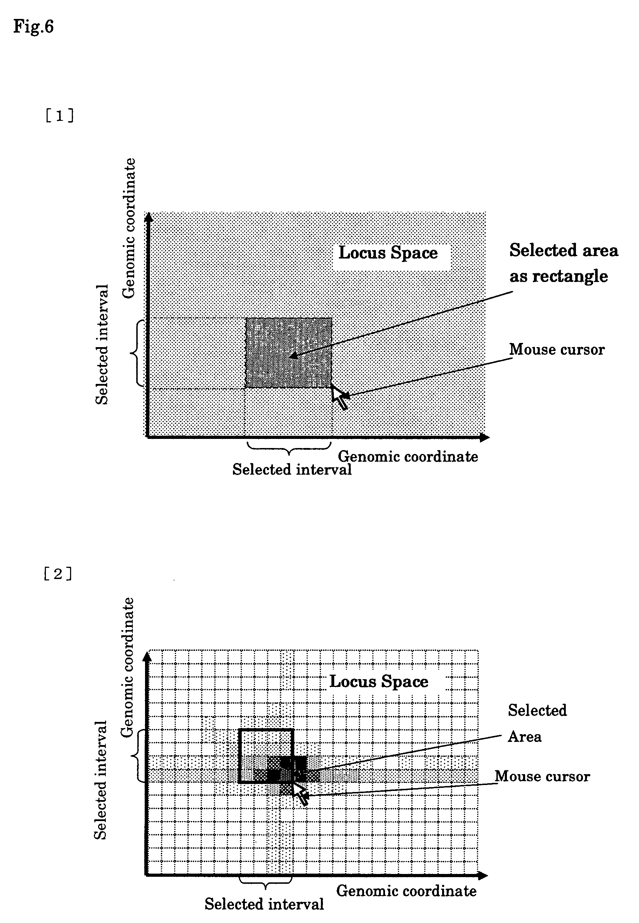

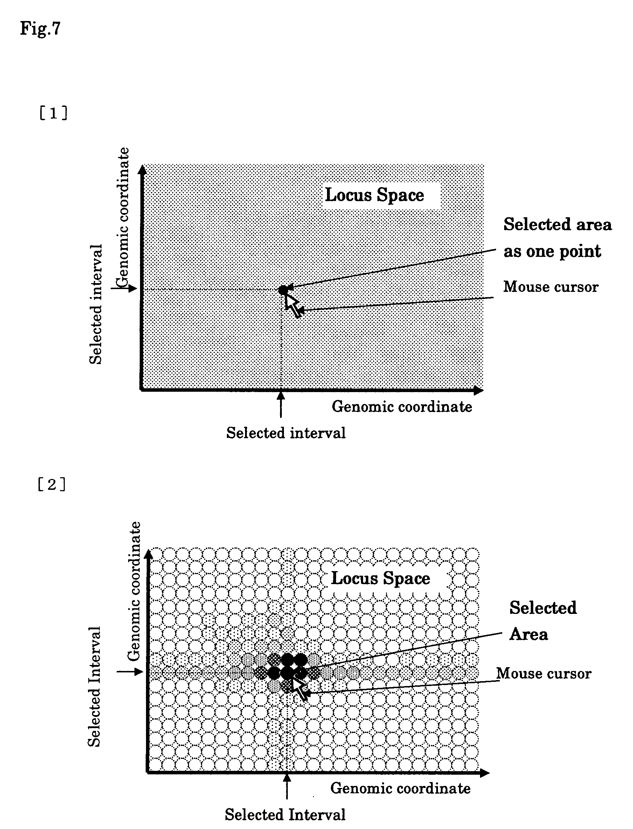

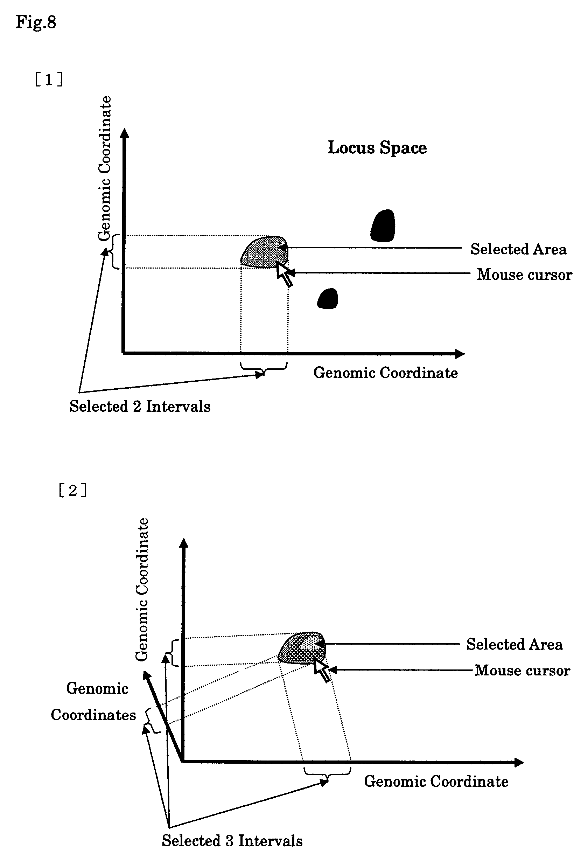

"Area" is a partial space in a gene locus space which can be selected by a user by operating an input device such as a mouse. Examples of the selection of an area include where a rectangular interval is selected by dragging a mouse as shown in Fig. 6 [1,2], or where a point in a locus space is selected by a mouse click in Fig.7[1,2]. As shown in Fig. 8, further example include where a degree of correlation between phenotypes and marker alleles is displayed in locus space, presenting only areas each of which gives a degree of correlation above a certain value. An area is selected by a user with a click of a mouse. In Fig. 8 [1], three areas are indicated each of which gives a degree of correlation above a certain value in a two-dimensional locus space, and one of the areas is selected by a click of a mouse. Fig. 8 [2] depicts an example where a single area gives a degree of correlation above a certain value in a three-dimensional locus space, and the area is selected by a click of a mouse.

"An interval corresponding to an area" is a segment interval projected geometrically from the area to a genomic coordinate axis, as shown in Figs. 6 [1,2] where the selected area as a rectangle. As shown in Figs. 7 [1,2], where an area selected is a point, the definition means an interval consisting of a point which is drawn geometrically perpendicular to each genomic coordinate axis from said area. For areas in which the degree of correlation in locus space is beyond a certain value, as shown in Fig. 8, the definition means a segment interval geometrically projected to each genomic coordinate axis from said area.

"Select simultaneously all of intervals corresponding to an area" is to determine automatically each intervals on each coordinate corresponding to the selected area.

"Database" is a means to store data. Any data storage devices may be used as long as they are readable and writable by a computer. A hard disk, DVD, memory and the like are suitably used. Relational database management software such as ORACLE and SQL Server may also be suitably employed. A file system is also suitably used as a database.

"Record" is a unit for handling data stored in a database. As a record, a file in a file system, a record in a relational database, an object in an object-oriented database and the like are suitably used. Data treatable as a single object by using a computer may sometimes be referred to as a record in the specification.

"Local computer" means a computer wherein a user, who views a trait map, can operate directly and/or a computer connected to a display or a monitor which can be directly watched by a user.

"Remote computer" means a computer which communicates with a local computer in this system, and is composed of one or more computers. A remote computer may be located at one site, or may be located at two or more sites.

As media to store a program, any media can be used so long as the media are readable by a computer. For example, memory, flash memory, hard disk, CD-ROM, DVD, MO, IC memory can be suitably used.

An example of achieving a system for an analysis of a trait map by using a computer will be explained below. However, the present invention is not limited to the example.

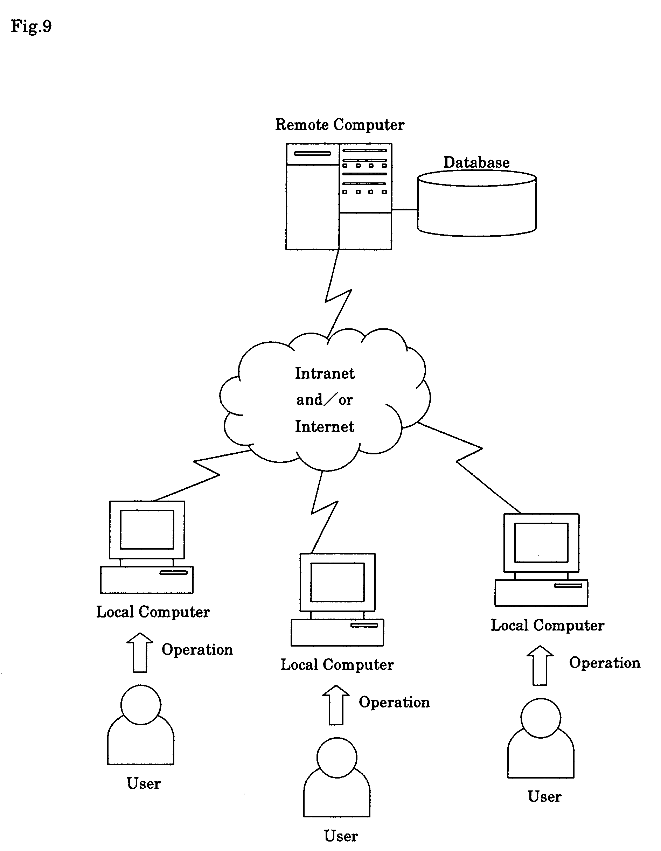

Fig. 9 shows an organization of computers according to the present system. Each user is able to operate a local computer directly by using a mouse and a keyboard. As a local computer, a commercially available notebook computer or desktop personal computer can be suitably used. A local computer displays information on a monitor connected to the local computer.

A local computer is connected to a remote computer via internet and/or intranet so as to enable communication with each other. A remote computer can access to a database and process data based on records in the database.

Programs such as a program for input and a program for presentation used in the present system are stored in a storage device of a remote computer.

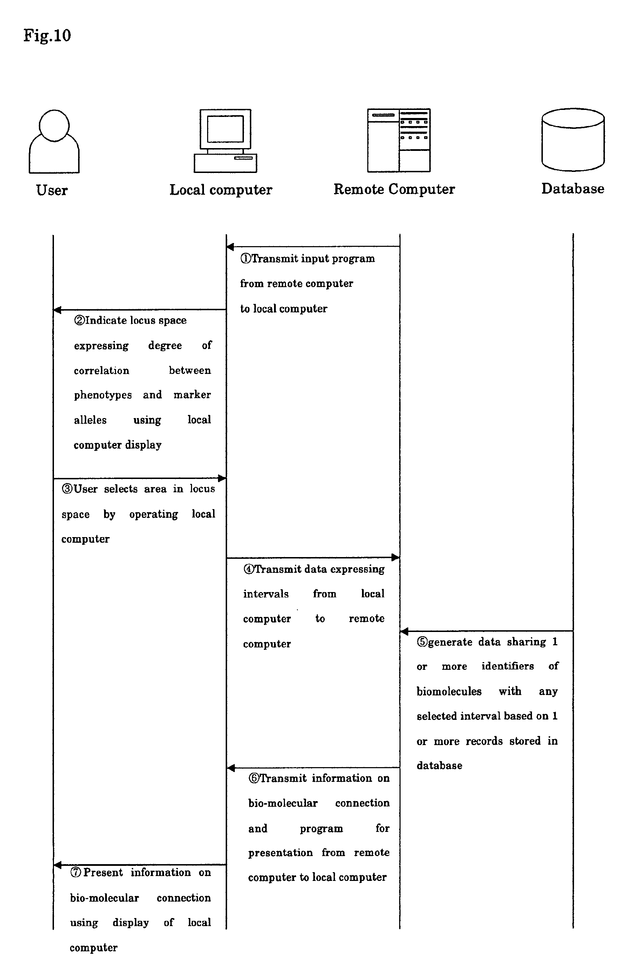

Fig. 10 shows a flow of information ( data and programs ) when the present invention is carried out by using the present system. The figure indicates an order of processes by a top-to-bottom order. First, a process of transmitting an input program from a remote computer to a local computer in ① is carried out. This process is started by a transmit request in an http protocol or an https protocol from the local computer side. The remote computer reads out necessary data for a trait map from a database and transmits said data to a local computer together with a program for input.

The program for input is mounted using HTML (Hyper Text Markup Language) and is operated on a web browser on a local computer. If necessary, it is possible to improve operationality of a user by employing a script program and/or an applet and/or a plug-in as a supplementary program on the local computer. When Active X control and plug-in are used on a web browser, it is preferred that these supplementary programs are installed in the local computer beforehand to download an HTML file received from the remote computer. Both HTML file and supplementary programs play a role as the program for input together.

A trait map is then presented by using a display or a monitor of the local computer as shown in Fig.10 ②. By assigning genomic coordinates to each axis of a two- or three-dimensional orthogonal coordinates system, a gene locus space is presented, and further a degree of correlation between phenotypes and marker alleles in the gene locus space is displayed. Thus, a trait map is presented to a user.

In Fig.10 ③, a user is able to select an area in a gene locus space by operations such as a click of a peak or a drag of a rectangular area with a mouse on a trait map displayed in the gene locus space.

Each interval corresponding to the selected area is calculated geometrically. When a two-dimensional orthogonal coordinates system is applied, the calculation enables selection of two intervals by a single mouse operation. When a three-dimensional orthogonal coordinate system is applied, the calculation enables selection of three intervals by a single mouse operation. In Fig.10 ④, an example is shown wherein the aforementioned calculation is carried out by the local computer, and data representing the calculated intervals are transmitted from the local computer to the remote computer. As an alternative method, information specifying an area is first transmitted from the local computer to the remote computer, and then the intervals are calculated on the remote computer.

In Fig.10 ⑤, data which share one or more names of bio-molecules with all of the selected intervals are generated based on one or more records stored in the database, which is referred to as "information on bio-molecular connection."

This process is carried out in the remote computer as follows. It is preferable to mount a program so as to first search from the database identifiers of genes whose gene loci exist in each interval, and then search a record from a database that shares one or more identifiers of genes with all of the selected intervals.

As another example of implementation, information on bio-molecular connection is generated beforehand by the aforementioned process for each of the areas and stored in the database. When an area is selected by a local computer, the remote computer sends to the local computer the stored information corresponding to the area.

In Fig.10 ⑥, information on bio-molecular connection or a program for presentation are transmitted from a remote computer to a local computer. It is preferable to carry out this operation by using http and https protocols in cooperation with Fig.2 ④. As another example of implementation, transmission may be performed as an e-mail using an smtp protocol.

In Fig.10 ⑦, information on bio-molecular connection is finally presented to a user using a display or a monitor of the local computer. A user is able to view a relation between genes whose loci exist in each selected interval by viewing the information on bio-molecular connection.

This information on bio-molecular connection is helpful for the following interpretation by a user.

Since epistasis (a combination effect of multiple genes) is observed in the selected multiple intervals in the trait map, it is expected that some sorts of mechanism which induces the combination effect of certain genes whose gene loci exist in each of the intervals. Therefore, once a common feature of genes in each interval is found, the feature will be helpful for a user to estimate the aforementioned mechanism. The information on bio-molecular connection is a datum that shares at least 1 or more identifiers of genes with all of the above intervals and may most likely be information expressing a common feature in genes in each interval, and accordingly, a user may view the information with expectation that the information may be helpful for deduction of the aforementioned mechanism.

Furthermore, by repeating the process of Fig.10 ③∼⑦, a user is able to view each peak observed on a trait map under a simple operation successively in connection with molecular level information, thereby reference information is obtainable from the present system which is used for selection of candidate causative genes of the trait.

The process of "generation of data sharing one or more identifiers of bio-molecules with all of selected intervals based on one or more records stored in a database" will be explained in details. Locus of each gene on genomic coordinates is stored beforehand in a database so that an identifier of a gene existing in the interval can be readily searched for any intervals on the genomic coordinates.

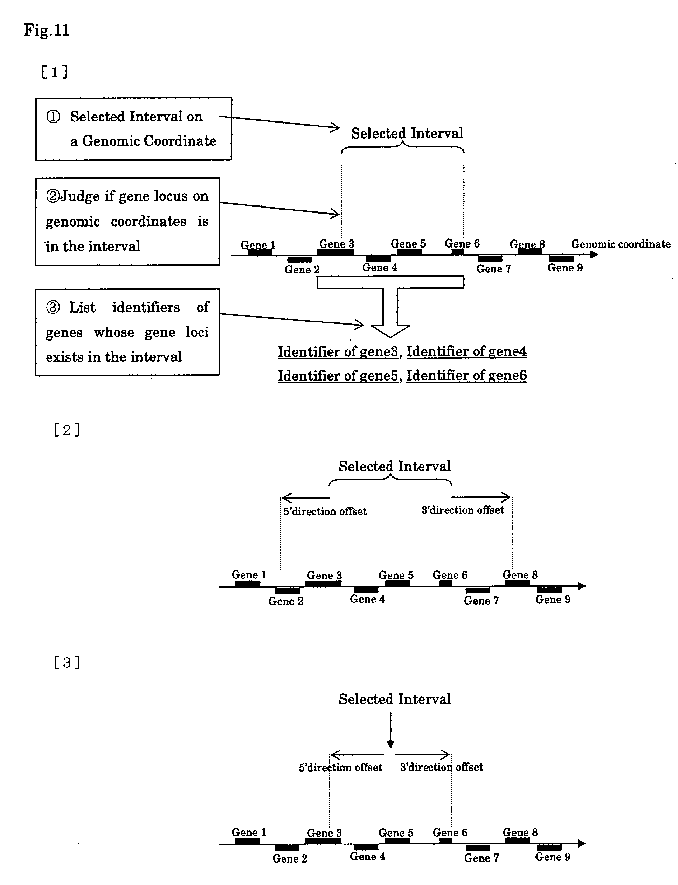

Fig. 11 depicts a method for selection of identifiers of genes whose gene loci exist in a selected interval. For an interval selected by a user, it is possible to search and list identifiers of genes loci of which exist in the interval in the database (Fig. 11[1]). As an alternative method, a search for the identifiers may be conducted on an interval expanded with offsets in the 5' direction and the 3' direction from the selected interval (Fig. 11[2]). Furthermore, when the selected interval has no width wherein the starting point and the end point overlap, it is necessary to search the identifiers by applying appropriate offsets (Fig. 11[3]). An offset may be often applied to have a range of several kilobase to several megabase, however, a smaller offset or a larger offset may also be applied. It is preferable to apply an offset by referring to a width of a peak in a trait map, which is most preferably be applied so as to be appropriately modifiable by a user.

"Information on bio-molecular connection" is a datum which shares one or more identifiers of bio-molecules with all of the selected intervals. For simplification, a specific example is given for explanation.
Case : "Two intervals, i.e., X and Y, are selected, four identifiers of genes, i.e., GX1, GX2, GX3, and GX4, are searched by using the X interval, and three identifiers, i.e., GY1, GY2, and GY3 are searched by using the Y interval."

For the aforementioned case, a database search is carried out by applying the following search query on the remote computer.
Search query: ("GX1" or "GX2" or "GX3" or "GX4") and ("GY1" or "GY2" or "GY3")

The meaning of this search query is to command a search for a record which contains at least one of GX1 to GX4 together with at least one of GY1 to GY3. As a result, for example, a record wherein "GX1 activates FK5, and the activated FK5 inhibits the activity of GY2" is assumed to be found. In this above case, the identifier of bio-molecule "GX1" exists both in this record and in the interval X, and therefore, it can be understood that this record and the interval X share the single identifier of the bio-molecule "GX1." Since "GY2" exists both in this record and in the interval Y, it can also be understood that "this record and the interval Y share the single identifier of the bio-molecule "GY2."

The above results can be summarized in that "this record is a datum which shares one or more identifiers of bio-molecules in all of selected intervals (interval X and interval Y)." The datum is referred to as "information on bio-molecular connection" in the specification. This case shows an example wherein information on bio-molecular connection is directly generated from a single record stored in a database. When two or more records are found which satisfy the aforementioned query, the result can be treated as generation of a single datum containing information on bio-molecular connection from those records. Thus, by the aforementioned methods, it is possible to search a datum that shares one or more gene identifiers in all of selected intervals (i.e., both of interval X and interval Y).

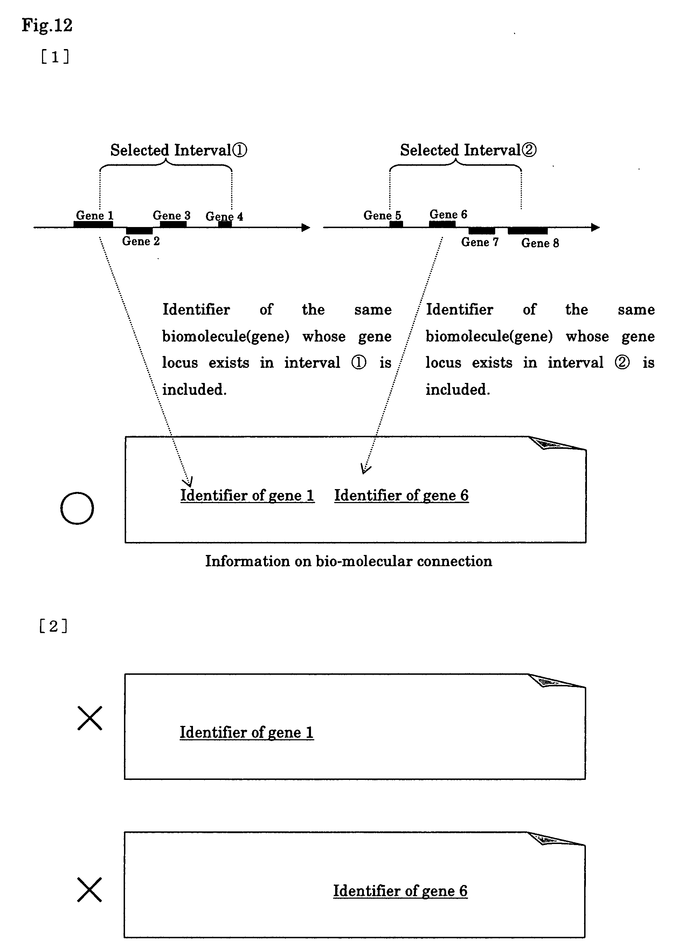

Fig. 12 illustrates the definition of the information on bio-molecular connection. Information on bio-molecular connection in Fig. 12 [1] includes an identifier of a bio-molecule (gene 1) whose gene locus exists in the selected interval ①, and also includes an identifier of a bio-molecule (gene 6) whose gene locus exists in the interval ②, and therefore, it satisfies a condition of "data that shares one or more identifiers of bio-molecules with all of selected areas."

On the other hand, each of two data examples shown in Fig. 12 [2] shares one or more identifiers of bio-molecules with one interval, however, fails to share the identifiers with the other interval. Consequently, each of these data is not the information on bio-molecular connection in the specification.

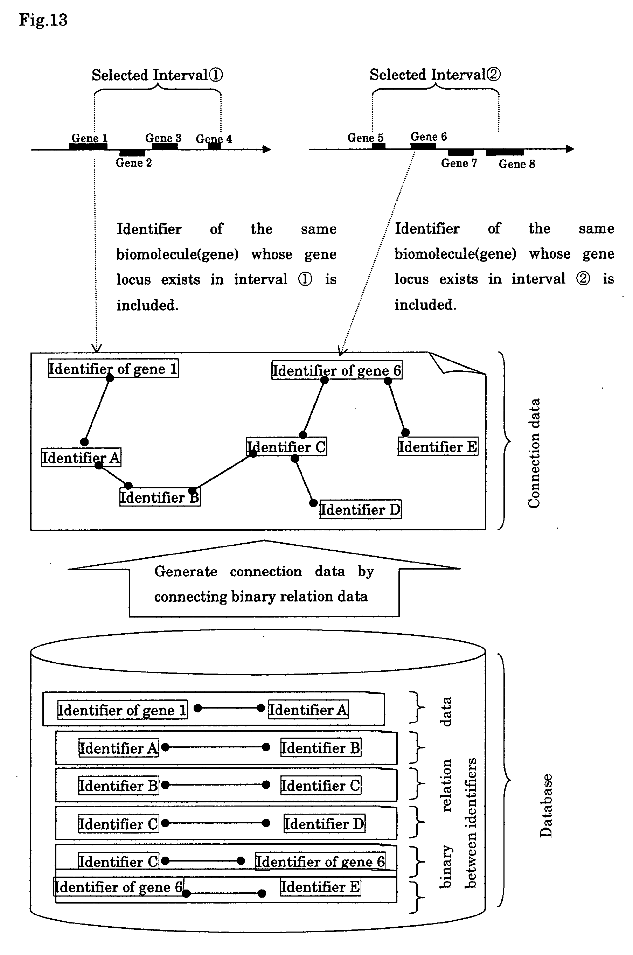

"Connection datum" is a graph wherein identifiers are used as nodes, which indicates relations between objects represented by those identifiers. Fig. 13 depicts an explanation on connection datum. The connection datum in the figure is a graph in which the identifier of gene 1 and the identifier of gene 6 are connected with a node and an edge, which expresses a relation between gene 1 and gene 6. For example, a series of a cascade can be represented by a graph of Fig. 13, wherein "a product of transcription from gene 1 (Identifier A) is translated to give protein B (Identifier B), protein B phosphorylates protein C (Identifier C), the phosphorylated protein C starts transcription of gene 6 which results in increase of an amount of a product of transcription of gene 6 (Identifier E), whilst protein D (Identifier D) connects with protein C to suppress transcription of gene 6". Since this graph shares one or more identifiers of bio-molecules with both of selected intervals ① and ②, this graph is also recognized as "information on bio-molecular connection."

The connection data can be generated deductively by connecting binary relation data between identifiers stored in a database. In the example shown in Fig. 13, a record which directly connects the identifier of gene 1 and the identifier of gene 6 by a binary relation does not exist in the database. However, by deductively connecting binary relation data which are stored in other records, the connection data shown in Fig. 13 can be generated. As a connecting method, an algorism is preferably used which comprises the steps of generating an adjacent matrix from binary relation data, generating a tree from the identifier of gene 1to the designated stratum based on the adjacent matrix, and from this tree obtaining all possible connection data between the identifier of gene 1 and the identifier of gene 6 (Graph Theory for Programmers, by V.N.Kasyanov and V.A. Evstigneev, Kluwer Academic Publishers, 2000). However, the connection method is not limited to the above exemplified method, and any deductive algorithm may be used.

### Examples

### Example 1

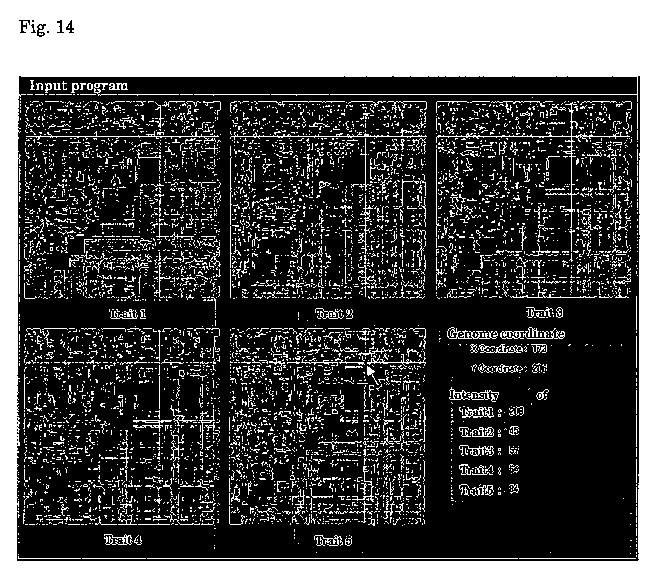

As an example of an input program which is able to simultaneously select two or more intervals, an example is shown in Fig. 14 wherein a trait map based on a circadian rhythm of mice is displayed. Five kinds of traits which reflect a circadian rhythm were measured to make trait maps, and the five trait maps are displayed in the window of the input program. When a mouse cursor is moved on any one of the trait maps, the position is also indicated with a white cross on the other four trait maps, and at the same time, a value showing the position on the genomic coordinate and a value indicating degree of correlation in each trait map are shown on the bottom right. When a peak is clicked on the trait map, two intervals on the genomic coordinates can be simultaneously selected, and information on bio-molecular connection is shown on a different window.

### Example 2

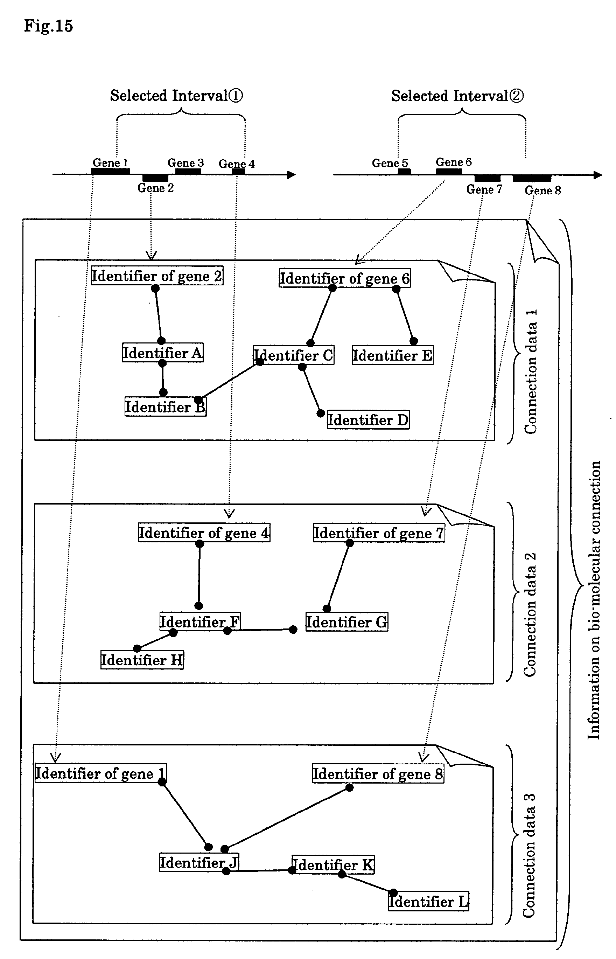

In this example, an example is shown wherein information on bio-molecular connection consisting of two or more connection data is displayed by a program for presentation. In Fig. 15, 3 connection data which share one or more identifiers of biomolecules with both of the intervals ① and ② are generated, a single information on bio-molecular connection consisting of these three connection data as a whole is generated.

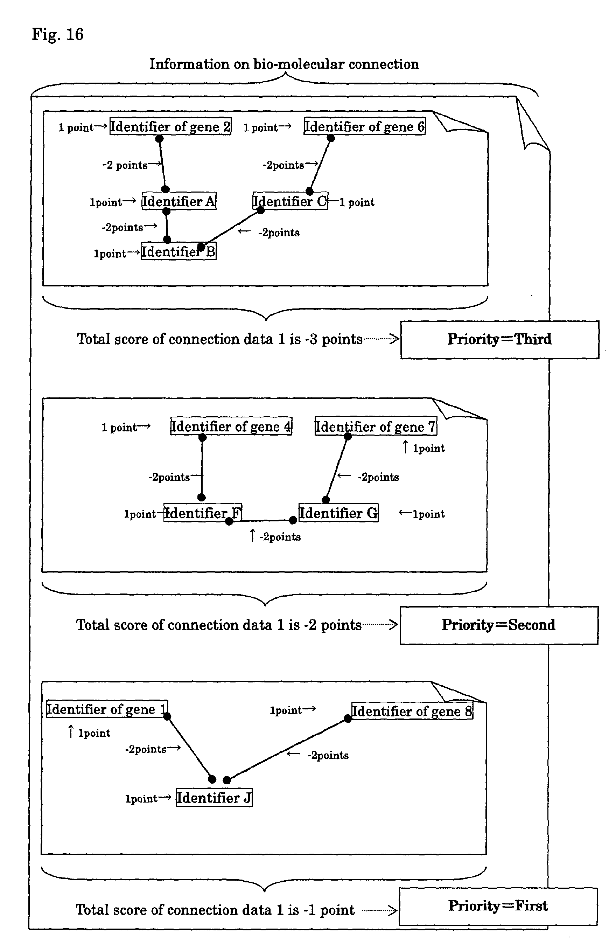

In Fig. 16 shows a procedure which gives a priority to these three connection data. Scores are first assigned to each identifier and each edge between the identifiers. Here in the example, point 1 is assigned commonly to an identifier and point -2 is assigned commonly to an edge for easy understanding. However, the method of the present invention is not limited to this particular assigning method, and assignment based on various criteria may be applied.

Then, in each of the connection data, a total score as explained below is calculated. A graph is traced from the identifier which is shared by the connection data and interval ① toward the identifier which is shared by the connection data and interval ②, and then a sum of the scores assigned to the identifiers and the edges which are passed through. Then, a total score based on a tracking way that gives the highest total score is appointed as the total score of the connection data. However, a total sum with the lowest score may sometimes be appointed to the total score depending on a method of score assignment. In Fig. 16, the total score of the connection data 1 is -3 points, the total score of the connection data 2 is -2 points, and the total score of the connection data 3 is -1 point. In this example, those with higher total scores can be viewed by a user preferentially. Accordingly, the order of highest priority is in the order of the connection data 3, the connection data 2, and the connection data 1.

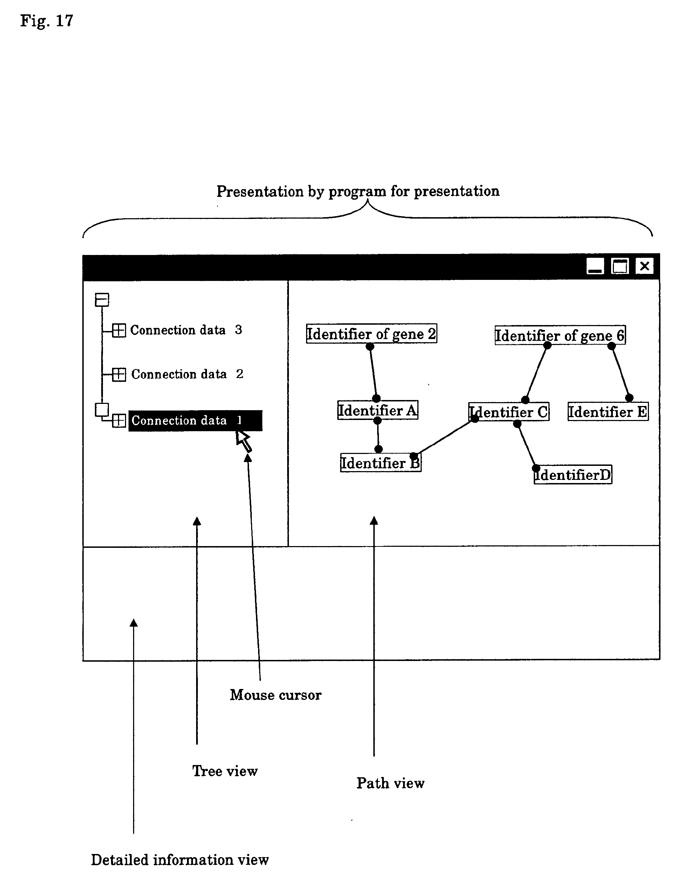

Fig. 17 is an example which relates to "a program for presentation wherein a user can select each of connection data by displaying the connection in the order of priority and view the selected connection data." A user interface of the program for presentation shown in Fig. 17 consists of a tree view, a path view, and a detailed information view. In the tree view, the name of each of connection data is listed in the order of priority so as to be selected by a user. As shown in Fig. 17, when the connection data 1 is selected by a mouse click, a graph of the connection data 1 is displayed in the path view so as to be viewed by a user.

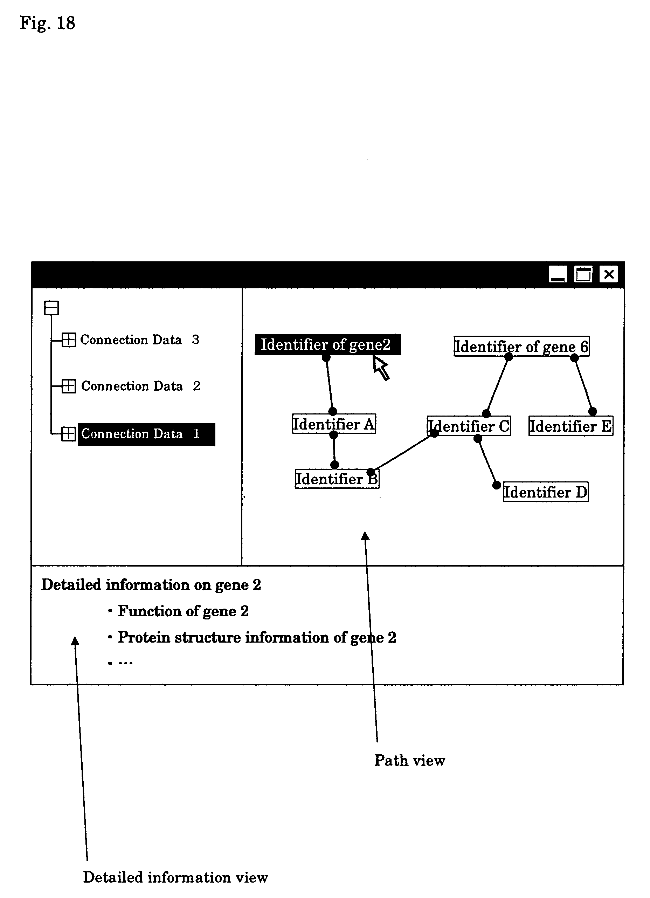

When a character string representing an identifier of gene 2, which is displayed in the path view, is selected as shown in Fig. 18, a detailed information on gene 2 is displayed in the detailed information view. An identifier drawn on a display or a monitor by an input program or an output program is referred to as "a character string representing an identifier" in the specification.

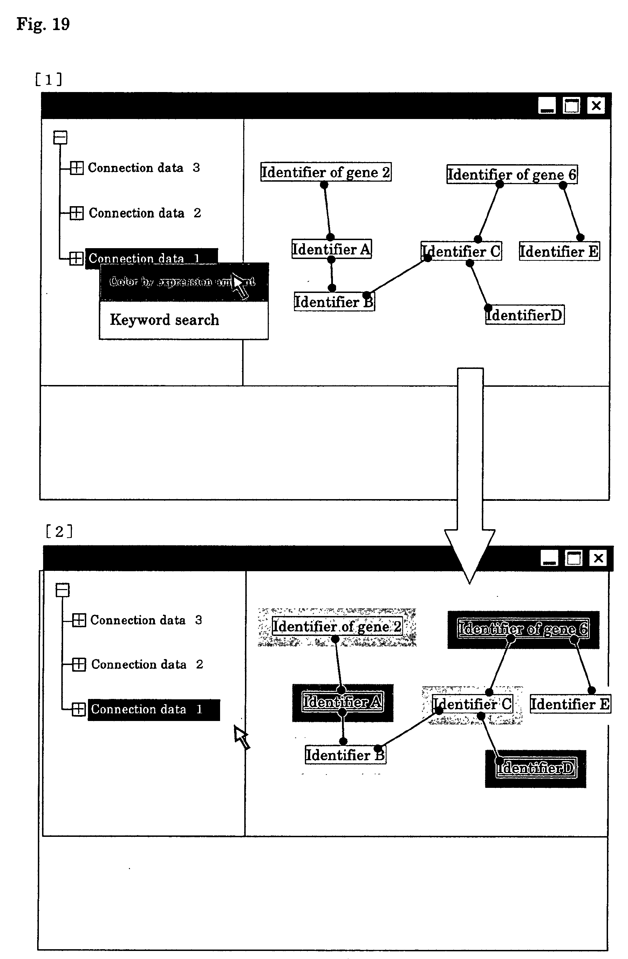

Fig.19 is an example relating to "a program for presentation by which a color of a character string representing an identifier of a bio-molecule or a background color of the character string is displayed depending on the intracellular expression amount in messenger RNA with the identifier of the bio-molecule." As shown in Fig. 19 [1], a submenu is displayed by a right click of a mouse, and select "coloring by expression amount" from the submenu. Then, as shown in Fig. 19 [2], when the expression amount of the gene corresponding to the identifier on the path view is recorded in the database, a background color of the character string representing the identifier is displayed depending on the expression amount. As data for expression amount, data measured by DNA microarray may suitably be used. For the coloring, a method of adjusting brightness of colors depending on degree of a change can be suitably used, for example, black for those with no change in expression amount, red for those with increased expression amount, green for those with decreased expression amount.

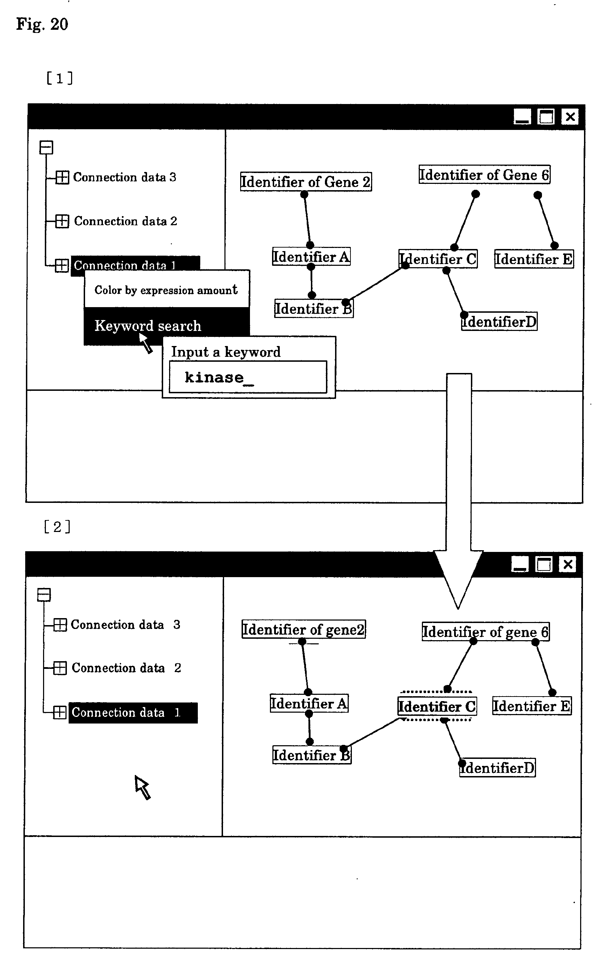

Fig. 20 is an example relating to "a program for presentation to indicate with highlight a character string representing an identifier of a bio-molecule which is hit by a keyword search" after carrying out a keyword search during the viewing of the information on bio-molecular connection by a program for presentation. As shown in Fig. 20 [1], a submenu is displayed by a right click of a mouse and "keyword search" is selected from the submenu, thereby the submenu is displayed for inputting a keyword. When a keyword "kinase" is input and a search is carried out, an identifier of a bio-molecule to which said keyword is matched in the database (identifier C in Fig. 20 [2]) is displayed with highlight. In this example, a character string representing the identifier is displayed in bold face and flashed for a way of display with highlight. However, a method of display with highlight is not limited to this example, and any methods may be used as long as they are sufficiently noticeable so as to draw a user's attention. For example, the character string may become noticeable by any one or combination of boldface, flashing, blinking, reflection, underline, Italic, or enlargement.

According to the present invention, an information system is first provided which enables analysis of a trait map in connection with a molecular level knowledge. For many peaks on a trait map, the present system enables an easy and rapid operation of judgment of whether or not each of the peaks is important by matching peak with molecular level knowledge, thereby selection work of candidate causative genes of the trait is easily carried out.

More specifically, by a method of the present invention, a viewer of a trait map can search and view candidate genes for a cause of the trait by a simple operation from a database. Furthermore, the viewer of the trait map can select candidate causative genes by connecting the trait map to molecular level genes by an interactive operation, thereby a lot of labor for an analysis of a trait map can be reduced, and moreover, many researchers can progressively carry out investigation by utilizing a trait map.

Moreover, by the aforementioned method, for many peaks with high degree of correlation between phenotypes and marker alleles found in a trait map, each peak is easily selected and analyzed by an interactive operation, and a viewer of the trait map can search and view the information on candidate causative genes of the trait by a simple operation. In particular, plural gene loci are required to be selected for an analysis considering epistasis, and it is much troublesome for a viewer to select each interval on the genomic coordinates individually where the each gene locus exists. By the method of the present invention, a viewer can select plural intervals on the genomic coordinates simultaneously and obtain molecular level information immediately by the aforementioned method. By applying the aforementioned method in a network environment such as internet or intranet, many researchers can utilize the analytical system of a trait map in their own laboratories, thereby information on necessary genes for analysis can be controlled centrally at one site.

## Claims

1. A method which provides a user who operates a computer with information on bio-molecular connection, the method comprises:
(1) step wherein the user selects two or more intervals on genomic coordinates by a computer operation;
(2) step of generating a datum which shares one or more identifiers of bio-molecules with all of the selected intervals in step (1) based on one or more records stored in a database; and
(3) step of providing the user with the generated datum as information on bio-molecular connection.

2. The method according to claim 1, wherein the computer is a local computer in an organization where multiple computers are connected by a network.

3. The method according to claim 2, wherein an input program which enables the user to select simultaneously the intervals in step (1) is used in the local computer.

4. The method according to claim 3, wherein a gene locus space is displayed by assigning genomic coordinates to each axis of a two- or three-dimensional orthogonal coordinates system, and an input program enables the user to select simultaneously all of intervals corresponding to an area in the locus space by selecting the area on the display.

5. The method according to claim 4, wherein a degree of correlation between phenotypes and marker alleles is displayed in the gene locus space.

6. The method according to any one of claims 1 to 5, wherein the information on bio-molecular connection contains one or more connection data.

7. The method according to claim 6, wherein a program for presentation is used which enables the user to select each of the connection data by displaying two or more connection data in an order of priority in step (3) and enables the user to view the selected connection data.

8. The method according to any one of claims 1 to 7, wherein a program for presentation is used by which a color of a character string representing an identifier of a bio-molecule or a background color of the character string is displayed depending on an intracellular expression amount of a messenger RNA of the identifier of the bio-molecule in step (3).

9. The method according to claim 7 or claim 8, wherein a program for presentation displays a character string of an identifier of a bio-molecule with highlight which represents the identifier of the bio-molecule hit in a keyword search or a homology search in step (3).

10. A program and/or a media which stores a program used to carry out the method according to any one of claims 1 to 9 by computers.

11. A computer and/or a database used to carry out the method according to any one of claims 1 to 9.
